# EUROPEAN PATENT SPECIFICATION

(11) **EP 2 349 204 B1**
(45) Date of publication and mention of the grant of the patent: **11.04.2018**
(21) Application number: 09783687.8
(22) Date of filing: 02.10.2009
(51) Int. Cl.: A61K 9/00, A61K 9/16

(54) **INHALABLE PARTICLES COMPRISING TIOTROPIUM**
TIOTROPIUM UMFASSENDE INHALIERBARE TEILCHEN
PARTICULES INHALABLES CONTENANT DU TIOTROPIUM

(30) Priority: 02.10.2008 EP 08382040; 09.10.2008 US 104113 P
(43) Date of publication of application: 03.08.2011
(73) Proprietor: Laboratorios Liconsa, S.A., 08028 Barcelona (ES)
(72) Inventor: AMIGHI, Karim, 1150 Brussels (BE); SERENO GUERRA, Antonio, 1820 Melsbroeck (BE)
(74) Representative: Schön, Christoph
(86) International application number: PCT/EP2009/062821
(87) International publication number: WO 2010/037845

(56) References cited:
- EP-A- 1 508 330
- WO-A-2007/075858
- WO-A-2009/007687
- WO-A2-2009/095681
- US-A1- 2002 081 266
- US-A1- 2004 042 970
- MALCOLMSON R J ET AL: "DRY POWDER FORMULATIONS FOR PULMONARY DELIVERY" PHARMACEUTICAL SCIENCE AND TECHNOLOGY TODAY, ELSEVIER TRENDS JOURNALS, CAMBRIDGE, GB, vol. 1, no. 9, 1 December 1998 (1998-12-01), pages 394-398, XP001205507 ISSN: 1461-5347

## Description

The present invention relates to inhalable particles comprising a stabilized amorphous form of tiotropium with a stabilizing agent, whereinthese particles are mixed with one or more coarse excipients. It also relates to a pharmaceutical composition comprising them, to a process for their preparation, and to their use in the treatment of asthma or chronic obstructive pulmonary disease (COPD).

### BACKGROUND ART

Tiotropium bromide is a muscarinic receptor antagonist with highly effective anticholinergic effect used as long active bronchodilator. It was first disclosed in the European Patent Application EP 418716 and has the following chemical structure:

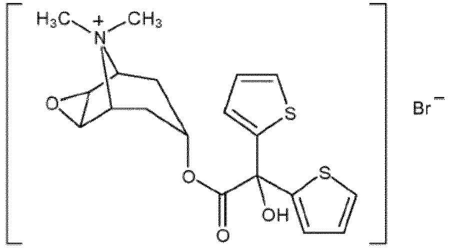

Tiotropium bromide is used in the treatment of respiratory diseases, in particular chronic obstructive pulmonary disease (COPD) and asthma.

Different salts of this product (chloride, bromide, iodide, etc.) as well as different crystalline forms thereof are known.

For the treatment of respiratory diseases such as asthma or COPD, it is useful to administer the active substance by inhalation. In comparison to other inhalers, dry powder inhalers (DPI) offer flexibility in terms of nominal dose range, that is the amount of active substance that can be administered in a single inhalation, this making them especially interesting as an administration tool. Thus, the use of inhalable powders containing active substances to be administered with a DPI is of particular importance.

The active compound, in order to be able to pass into the lungs must be inhalable; that is, it must be present in particles of size about 1 to 10 µm. Microfine particles of this size can be obtained, for example, by micronization, controlled precipitation from suitable solvents or by spray-drying if the process conditions are suitably selected, controlled and carried out.

Traditionally, dry powders have been formulated as micronized drug blended with a coarse carrier particle, typically lactose. Dry powder formulations of micronized tiotropium blended with a coarse carrier particle have also been described in the state of the art. For example, EP 1292281 discloses a preparation process including micronization comprising several mixing steps of the excipients. However, micronization is a poorly reproducible method and highly affected by small variability on the starting material. Moreover, such compositions, including the marketed medicament of Boehringer Ingelheim, Spiriva®, have been proved to show poor performance in terms of dispersibility and fine particle fraction. Moreover the process for its preparation shows the difficulty to obtain homogeneous mixtures between tiotropium, the fine and the coarse carriers since long and multiple blending steps are needed. In other example, EP1508330 discloses a capsule which contain tiotropium mixed with an acceptable excipient, preferably contains crystalline tiotropium bromide monohydrate. However, the stabilizing agent is mixed mechanically with amorphous tiotropium particles, this means that there is not a straight contact between the stabilizing agent and amorphous tiotropium.

Thus, there is a need for improvement of inhalable dry powder compositions of tiotropium which avoid the disadvantages of the prior art formulations and show improvements concerning the homogeneity of the powder mixtures, the powder flowability and /or the powder dispersion, and aerodynamic properties.

### SUMMARY OF THE INVENTION

Inventors have found a stabilized anhydrous amorphous form of tiotropium with a stabilizing agent that give rise to inhalable powder compositions showing a high degree of homogeneity, and minor fluctuations in the dispersion properties. These factors are crucial in ensuring that the inhalable proportion of active substance is released reproducibly in constant amounts and with the lowest possible variability. The compositions of the invention also have improved flow properties, a high dispersibility and an enhanced Fine Particle Fraction (FPF) by using an appropriate dry powder inhaler, as it will be shown in the examples.

Furthermore, unlike what occurs in case of preparing inhalable particles containing a crystalline form, where accurate and laborious process conditions to obtain the desired form are needed, the inhalable particles of the invention can be obtained by a simple and fast process since they are in an amorphous form.

Therefore, a first aspect of the present invention refers to inhalable particles comprising a stabilized anhydrous amorphous form of tiotropium with a stabilizing agent, which is lactose, wherein the stabilized amorphous form of tiotropium with the stabilizing agent comprises a matrix in which tiotropium is dispersed in a molecular state or wherein the stabilized amorphous form of tiotropium with the stabilizing agent comprises amorphous tiotropium dispersed at the surface of the stabilizing agent, and wherein the particles are mixed with one or more coarse excipients having a mean particle size of 15 to 250 µm. The inhalable particles may comprise a matrix in which tiotropium is intimately dispersed in a molecular state, or amorphous tiotropium dispersed at the surface of the stabilizing agent, i.e. the particles of the stabilizing agent are coated by a thin layer of amorphous tiotropium.

In drugs such as tiotropium, which show a particularly high efficacy, only small amounts of the active substance are needed in each single dose to achieve the desired therapeutic effect. As a consequence, the active substance has to be diluted with one or more pharmacologically inactive substances (suitable excipients) in order to obtain flowable powders. The dilution must be such that the amount applied from the powder inhaler exactly contains the desired dose. These pharmacologically inactive excipients are not only used as diluents, but also for their ability of giving good flowability properties to the powder composition, facilitating thus the mixing process.

One approach used to enhance the powder flowability relates to the use of one or more coarse excipients. Such coarse excipients used as carriers must have a particle size which makes them not inhalable, since microfine particles tend to show strong adhesion and cohesion which in turn lead to poor flow properties and to powder aggregation. Thus, according to the present invention the inhalable particles of the invention are mixed with one or more coarse excipients having a mean particle size of 15 to 250 µm.

Therefore, according to the invention the inhalable particles comprising a stabilized amorphous form of tiotropium with a stabilizing agent, which is lactose, are mixed with one or more coarse excipients having a mean particle size of 15 to 250 µm. These particles will be also referred to herein as particles mixed with one or more coarse excipients.

It has been found that the stabilized amorphous form of tiotropium with a stabilizing agent of the present invention also allows to obtain dry powder formulations with acceptable flowability by using only fine ingredients. As it will be explained below in more detail, this can be achieved by preparing inhalable particles by a suitable drying-method starting from solutions of tiotropium and a stabilizing agent at an appropriate dilution.

The inhalable particles of the present invention as defined above may be administered to a patient suffering from a respiratory disease in the form of an appropriate pharmaceutical composition. Thus, another aspect of the present invention relates to pharmaceutical compositions comprising the inhalable particles of the present invention as defined above. These compositions will be further described below.

The inhalable particles of the invention can be conveniently prepared by an appropriate drying method from a tiotropium-stabilizing agent solution or suspension. Therefore, another aspect of the invention refers to a process for preparing inhalable particles comprising the following steps:
a) dissolving or dispersing the stabilizing agent in a volatile water miscible solvent optionally containing water, to form a solution or a suspension;
b) dissolving a tiotropium salt, or a solvate thereof, or any solid form thereof, in a volatile water miscible solvent optionally containing water;
c) mixing the solution of step b) and the solution or suspension of step a);
d) spray-drying the solution/suspension of step c) to obtain the desired particles; and
e) mixing the particles of step d) with one or more coarse excipients having a mean particle size of 15 to 250 µm.

As previously described, the compositions of the invention are useful for the treatment of certain respiratory diseases. Therefore, another aspect of the present invention relates to the inhalable particles as previously defined, for use in the treatment of asthma or chronic obstructive pulmonary disease (COPD). Thus, this aspect relates to the use of the inhalable particles as previously defined, for the manufacture of a medicament for the treatment of asthma or COPD, and may also be formulated as a method for the treatment of asthma or COPD comprising administering an effective amount of the previously defined inhalable particles of the invention, including the inhalable particles without coarse excipient, and the inhalable particles with one or more coarse excipients, in a subject in need thereof.

These aspects of the present invention will be further described in the detailed description section that follows. Unless otherwise defined, all technical and scientific terms used herein have the same meaning as commonly understood by one of ordinary skilled in the art to which this invention belongs. Throughout the description and claims the word "comprise" and its variations are not intended to exclude other technical features, additives, components, or steps.

### BRIEF DESCRIPTION OF THE DRAWINGS

FIG. 1 shows the PXRD patterns of untreated tiotropium bromide (A) and spray-dried tiotropium bromide after storage for 6 months at 25 °C (B).
FIG. 2 shows the PXRD patterns of spray-dried tiotropium bromide-lactose powder obtained from ethanol-water solutions just after spray-drying (A) and after storage for 6 months at 25 °C (B).
FIG. 3 shows SEM images for spray-dried powders obtained from tiotropium-lactose in solution at different magnifications.
FIG. 4 shows SEM images for spray-dried powders obtained from tiotropium-lactose in suspension at different magnifications.

### DETAILED DESCRIPTION OF THE INVENTION

According to the invention, a first aspect relates to inhalable particles comprising a stabilized anhydrous amorphous form of tiotropium with a stabilizing agent as defined above.

The term tiotropium refers to any pharmaceutically acceptable salt comprising tiotropium as the free ammonium cation and an anion as the counter-ion. Non-limiting tiotropium salts which may be used within the scope of the present invention are those compounds which contain, for example, chloride, bromide, iodide, methanesulphonate, para-toluenesulphonate, benzenesulphonate or methyl sulphate. According to the invention, the tiotropium present in the stabilized amorphous form is anhydrous. In a preferred embodiment, the inhalable particles of the invention comprise a stabilized amorphous form of a tiotropium salt with a stabilizing agent. In a more preferred embodiment, the tiotropium salt is tiotropium bromide. The term tiotropium base refers to tiotropium as the free ammonium cation.

The stabilizing agent as described herein, which is lactose, must be capable of stabilizing the active substance. Lactose is used in the compositions according to the invention in order to prevent or to slow down the physical transformation (i.e. from an amorphous to a crystalline form) and/or the chemical degradation (e.g. by hydrolysis, oxidation or any other degradation mechanism) of tiotropium. It means that the physical transformation of amorphous tiotropium or its chemical degradation during storage (e.g. at 25°C/60%RH, 30°C/65%RH or 40°C/75%RH) is slower when inhalable particles contained both a stabilizing agent, which is lactose, and amorphous tiotropium in their composition in comparison to inhalable particles containing amorphous tiotropium only.

According to the present invention the stabilizing agent is lactose (anhydrous or monohydrate).

Within the scope of the invention, the term "amorphous" means that the powdered formulation contains less than 10% crystalline fractions.

The term "inhalable" means that the particles are suitable for pulmonary administration. Inhalable particles can be dispersed and inhaled by means of an inhaler, so that the particles enter the lungs and are able to develop a systemic activity optionally through the alveoli. In one embodiment of the invention, the inhalable particles comprising the amorphous matrix of tiotropium and the stabilizing agent have a mean aerodynamic particle size of up to 10 µm. In a preferred embodiment, the mean aerodynamic particle size is in the range 0.5-6 µm.

In a preferred embodiment, the % tiotropium bromide in weight referred to the weight of tiotropium bromide and stabilizing agent is comprised between 0.1-10 %, preferably between 4-8%.

In the present invention, the inhalable particles comprise a stabilized amorphous form of tiotropium with a stabilizing agent. The term "stabilized amorphous form of tiotropium" as described in the invention refers to the compositions obtained by the production processes used in the scope of this invention to obtain inhalable particles containing a stabilizing agent and amorphous tiotropium.

The term "stabilized amorphous form" includes both particles, wherein the stabilized amorphous form of tiotropium with the stabilizing agent comprises:
a) a matrix in which tiotropium is intimately dispersed in a molecular state i.e. inhalable particles obtained by drying a solution containing both the stabilizing agent and tiotropium; and b) amorphous tiotropium dispersed at the surface of the stabilizing agent, i.e the stabilizing agent is coated by a thin layer of amorphous tiotropium, in that case the inhalable particles are obtained by drying a suspension of the stabilizing agent containing tiotropium in solution).

In both cases, the straight contact between the stabilizing agent and amorphous tiotropium (i.e. an intimate contact between molecules of the stabilizing agent and tiotropium, or an intimate contact between the stabilizing agent particles and the layer of amorphous tiotropium coating) is necessary to obtain a stabilized amorphous form of tiotropium. It means that a better stability of amorphous tiotropium is obtained when the inhalable particles are obtained by using productions processes as described in this invention, in comparison to production processes based on the use of a blending step only, in which the stabilizing agent is mixed mechanically with amorphous tiotropium particles (i.e. using an appropriate mixer for blending pharmaceutical powders).

In order to obtain inhalation compositions with good flow properties it is necessary to mix the inhalable particles comprising a stabilized amorphous form of tiotropium and a stabilizing agent with one or more coarse excipients.

Thus, depending on the ratio tiotropium-stabilizing agent, when the stabilized amorphous form of tiotropium with a stabilizing agent comprises a matrix in which tiotropium is intimately dispersed in a molecular state, the pharmaceutical compositions of the invention are prepared from the particles obtained after applying the suitable drying method conveniently mixed with one or more coarse excipients having a mean particle size of 15 to 250 µm.

When the stabilized amorphous form of tiotropium with a stabilizing agent comprises amorphous tiotropium dispersed at the surface of the stabilizing agent, the pharmaceutical compositions of the invention must be prepared from the particles obtained after applying the suitable drying method, wherein the particles are mixed with one or more coarse excipients having a mean particle size of 15 to 250 µm.

In a preferred embodiment of the invention, only one coarse excipient is used and it has preferably a mean particle size ranging from 50-150 µm.

The stabilizing agent, which is lactose, and the coarse excipients may be based on chemically identical or different substances. In a particular embodiment, the stabilizing agent and the coarse excipient comprise the same chemical compound. In a preferred embodiment, the stabilizing agent, as well as the coarse excipient, comprise lactose, optionally in the form of monohydrate.

The pharmaceutical compositions of the invention preferably comprise from 0.02 to 0.8 % of tiotropium base.

Unless stated otherwise herein, the percentages given within the scope of the present invention are always percent by weight.

Preferably, the compositions of the invention are in the form of a capsule for inhalation. The capsule may be formed of various materials, such as gelatine, cellulose derivatives, starch, starch derivatives, chitosan and synthetic plastics. In a preferred embodiment, the capsule is formed of cellulose derivatives. Non-limiting examples of such derivatives are hydroxypropylmethylcellulose (HPMC), hydroxypropylcellulose (HPC), methylcellulose, hydroxymethylcellulose or hydroxyethylcellulose. In a more preferred embodiment of the invention, the inhalation capsule comprises HPMC. In the most preferred embodiment, the HPMC capsules show a low residual humidity (water content below 4%).

The inhalable particles of the invention may be prepared either starting from a solution or a suspension of the stabilizing agent in a suitable solvent. An advantage of the present method is that the pharmaceutical compositions of the invention give neutral pH values after dissolution in water. This is very interesting in term of lung tolerance as the neutral pH values of the lungs should not be modified after inhalation.
In the case of starting from a solution of the stabilizing agent, the stabilizing agent is dissolved in a water miscible solvent containing water (solution of step a), and this solution is mixed with the solution obtained from tiotropium in the same solvent (solution of step b). In this case, as it will be obvious to a person skilled in the art, these steps may be carried out in any order in order to obtain a solution comprising tiotropium and the stabilizing agent.
After applying the suitable drying method, the resulting particles obtained comprise a matrix in which tiotropium is intimately dispersed in a molecular state in the stabilizing agent (i.e; a matrix of lactose containing tiotropium is formed) in order to obtain an efficient stabilization effect on the active in an amorphous form.

When the inhalable particles of the invention are prepared starting from a suspension of the stabilizing agent, the steps a) and b) are preferably carried out separately, and more preferably, the stabilizing agent is firstly dispersed and homogenized (for instance by using high speed or high pressure homogenization) in order to obtain an homogeneous suspension of the stabilizing agent, before adding the solution of the active ingredient (solution of step b). In this case, after applying the suitable drying method, the resulting particles comprise amorphous tiotropium dispersed at the surface of particles of the stabilizing agent. In this case, the stabilizing agent is preferably in the form of particles having a mean particle size of 1-9 µm.

The solvent system used for the preparation of the inhalable particles described above comprise a volatile water miscible solvent optionally containing water. As explained above, the presence of water gives rise to solutions of the stabilizing agent, whereas its absence gives rise to suspensions of said agent. In the case that a solution of the stabilizing agent is used, depending on the amount of the stabilizing agent used, appropriate water miscible solvent should be added in order to totally solubilize the stabilizing agent. On the other hand, the active ingredient is always dissolved. Preferably, the volatile water miscible solvent is a (C₁-C₄)-alcohol. The term (C₁-C₄)-alcohol refers to a linear or branched alkyl chain comprising from 1 to 4 carbon atoms and one or more hydroxyl groups. This term includes, without limitation, methanol, ethanol, propanol, isopropanol and butanol. In a preferred embodiment of the invention, the alcohol solvent used is ethanol or isopropanol.

The drying method used is spray-drying. This method allows to contribute to obtain a precise control of the mean particle size and particle size distribution, and also the improvement of the macroscopic powder properties such as flowability, and dispersibility. A typical Spray-Dryer allows the recovery of a range of particle size going from 0.5 to 30 µm. The lower limitation is given by the separation of the cyclone used: smaller particles can not be separated anymore and are going into the filter.

The described processes for the preparation of inhalable particles has the advantage that avoids micronization by using a more reproducible process, since micronization is a poorly reproducible method and highly dependent on the physical properties of the starting material. In the case of the present invention, the starting material (tiotropium compound) may be in any physical form, since it is firstly dissolved. Thus, the starting tiotropium compound may be any tiotropium salt or a solvate thereof, or any solid form thereof, including the racemate or any enantiomer thereof or mixtures thereof.

As mentioned above, the inhalable particles of the invention show an enhanced fine particle fraction. The term "fine particle fraction" (FPF) describes the inhalable part of a powder. The FPF is the dose (expressed in weight %) of particles having an aerodynamic diameter inferior to 5 µm in relation to the nominal dose (Fine particle dose/loaded dose x 100).The Fine Particle Dose (FPD) can be determined by the methods described in the USP and European Pharmacopoeia for the aerodynamic assessment of fine particle. In powder which is well dispersible the FPF is more than 20%, preferably more than 30%. The term "mean aerodynamic particle diameter" also known as mass median aerodynamic diameter (MMAD) indicates the aerodynamic particle size at which 50% of the particles of the powder have a smaller aerodynamic diameter.

### EXAMPLES

The following examples are provided for illustrative means, and are not meant to be limiting of the present invention.

### High Performance Liquid Chromatography (HPLC)

The tiotropium content determination in the spray-dried formulations and final tiotropium-lactose blends was carried out by HPLC.

The HPLC system consisted of a High Performance Liquid Chromatography system (HP 1200 series, Agilent technologies, Belgium), equipped with a quartenary pump, an autosampler and a diode array UV detector set at 238 nm. The separation system was a 125 mm x 4 mm stainless steel (5 µm particle size) reversed-phase C18 column (Alltima, Alltech, Belgium). Samples of 100 µl volume were injected. The temperature was set to 35 °C and the flow rate was 0.8 ml/min. The total run time was 10 minutes.
- *Buffer:* KH₂PO₄ 100 mM adjusted to pH 4 with o-phosphorique acid.
- *Mobile phase:* 80 vol. Phosphate buffer: 20 vol. acetonitrile.
- *Dilution phase:* 75 vol. water: 25 vol. methanol.

In table 1 are shown the standard solutions containing tiotropium.

### Standard solutions

S1: 2.5 mg Tiotropium Br monohydrate in 10 ml methanol (= 200 µg/ml de Tiotropium base)
S2: 1.0 ml of S1 solution in 25 ml of dilution phase (= 8 µg/ml)

**Table 1**

| **Standards** | **Vol S2 (ml)/ Total Vol. (ml)** | **Conc. of Tiotropium base (µg/ml)** |
|---|---|---|
| 1 | 0.250/100 | 0.02 |
| 2 | 0.250/50 | 0.04 |
| 3 | 0.200/20 | 0.08 |
| 4 | 0.400/20 | 0.16 |
| 5 | 1.0/20 | 0.40 |
| 6 | 2.0/20 | 0.80 |
| 7 | 4.0/20 | 1.60 |
| 8 | 5.0/20 | 2.00 |

### Preparation of stable tiotropium formulations by spray-drying

### a) Preparation of inhalable particles comprising tiotropium bromide and lactose microfine (Lactochem, Borculo; mean particle size of about 8 µm)

The formulations were prepared, at laboratory scale, by spray-drying using a Büchi Mini Spray Dryer B-191a (Büchi laboratory-Techniques, Switzerland). Different solution and suspensions were prepared in ethanol or isopropanol containing or not different amounts of water.

Firstly, lactose was dissolved or dispersed in the solvent medium in order to obtain solutions (in the presence of sufficient amounts of water) or suspensions (in the absence of water). Then, tiotropium bromide was dissolved in the solvent medium and the obtained solutions or suspension and solution were mixed. In the absence of water, lactose was firstly dispersed and homogenized (for instance by using high speed or high pressure homogenization) in order to obtain an homogeneous suspension, before adding the solution of tiotropium.

The preparations were then spray-dried with constant stirring. The following conditions were used during spray-drying: spraying air flow, 800 l/h; drying air flow, 35 m³/h; suspension feed rate, 3.5-4.0 g/min; nozzle size, 0.5 mm. The inlet temperature was established at 70° C or 120°C (in presence of water) and, in these conditions, the outlet temperature varied between 44° and 46° C or 65° and 68°C. The resultant powder was blown through the cyclone separator and collected in a container. Powders were stored in desiccators at different temperature conditions (25°C, 40°C and 60°C).

Following this process the following examples were obtained:

### Example 1: Tiotropium - lactose solution in ethanol - water

| | |
|---|---|
| Ethanol | 100 g |
| Water | 100 g |
| Lactose Microfine | 4.480g |
| Tiotropium bromide monohydrate | 0.360g |

### Example 2: Tiotropium - lactose solution in isopropanol - water

| | |
|---|---|
| Isopropanol | 100g |
| Water | 100g |
| Lactose Microfine | 4.480g |
| Tiotropium bromide monohydrate | 0.360g |

### Example 3: Tiotropium - lactose suspensions in ethanol

| | |
|---|---|
| Ethanol | 200g |
| Lactose Microfine | 4.480g |
| Tiotropium bromide monohydrate | 0.360g |

### Example 4: Tiotropium - lactose suspensions in isopropanol

| | |
|---|---|
| Isopropanol | 200g |
| Lactose Microfine | 4.480g |
| Tiotropium bromide monohydrate | 0.360g |

### Example 5: Tiotropium - lactose solution in ethanol - water

| | |
|---|---|
| Ethanol | 100 g |
| Water | 100 g |
| Lactose monohydrate 200 mesh (mean particle size of about 74 µm) | 4.480g |
| Tiotropium bromide monohydrate | 0.360g |

### Example 6: Tiotropium - lactose solution in isopropanol - water

| | |
|---|---|
| Isopropanol | 100g |
| Water | 100g |
| Lactose monohydrate 200 mesh (mean particle size of about 74 µm) | 4.480g |
| Tiotropium bromide monohydrate | 0.360g |

### Example 7: Tiotropium - lactose solution in ethanol - water

| | |
|---|---|
| Ethanol | 500 g |
| Water | 500 g |
| Lactose Microfine | 88.02g |
| Tiotropium bromide monohydrate | 0.360g |

### b) Preparation of inhalable particles obtained in step a) mixed with a coarse excipient: lactose monohydrate 200 mesh (mean particle size of about 74 um) or roller dried anhydrous lactose (mean particle size of about 120 um)

For this purpose, a laboratory scale well-known three-dimensional motion mixer: Turbula 2C (Bachofen AG, Switzerland) was used. A 50 mL non crosslinked polyethylene plastic vessel was filled to 50% of its inner volume and the blending (about 20 g) was done at mixing speeds of 46.2 rpm.

About 25% of lactose was added first to line the mixer bowl walls in order to reduce further drug adhesion. The spray-dried powder obtained in step a) was then added and mixed manually with lactose. Then the rest of lactose was added and mixing was carried out for about 30 minutes.

Example 1 was blended with the coarse excipient (monohydrate lactose and anhydrous lactose) to give rise to batches 1A (example 8) and 1B (example 9), respectively.

Example 3 was blended with the coarse excipient (monohydrate lactose and anhydrous lactose) to give rise to batches 3A (example 10) and 3B (example 11), respectively.

### Example 8: Blending operation (Turbula) - monohydrate lactose

| | |
|---|---|
| Spray-dried powder obtained as in step a) from Example 1 | 1.200 g |
| Lactose monohydrate (74 µm, DMV) | 20.810 g |
| Total weight | 22.010 g |

### Example 9: Blending operation (Turbula) - anhydrous lactose

| | |
|---|---|
| Spray-dried powder obtained as in step a) from Example 1 | 1.200 g |
| Lactose anhydrous (120 µm, DMV) | 20.810 g |
| Total weight | 22.010 g |

### Example 10: Blending operation (Turbula) - monohydrate lactose

| | |
|---|---|
| Spray-dried powder obtained as in step a) from Example 3 | 1.200 g |
| Lactose monohydrate (74 µm, DMV) | 20.810 g |
| Total weight | 22.010 g |

### Example 11: Blending operation (Turbula) - anhydrous lactose

| | |
|---|---|
| Spray-dried powder obtained as in step a) from Example 3 | 1.200 g |
| Lactose anhydrous (120 µm, DMV) | 20.810 g |
| Total weight | 22.010 g |

### Unit final composition of the previous examples

| | |
|---|---|
| Tiotropium (base) | 18 µg |
| Lactose monohydrate (stabilizing agent) | 0.2775 mg |
| Lactose monohydrate or anhydrous (coarse excipient) | 5.2025 mg |

In these examples, the final content of tiotropium bromide was around 0.39 % (around 0.33% for tiotropium base) in weight as referred to the total composition.

### X-ray Powder Diffraction (XRPD)

Powder X-Ray Diffraction (XRPD) patterns were obtained using a Siemens Diffractometer D5000 (Siemens, Germany), with a Cu line as the source of radiation (WL1 = 1.5406 A, WL2 = 1.54439 A), and standard runs using a 40 kV voltage, a 40 mA current and a scanning rate of 0.02°/min over a 2 θ range of 2° to 70°.

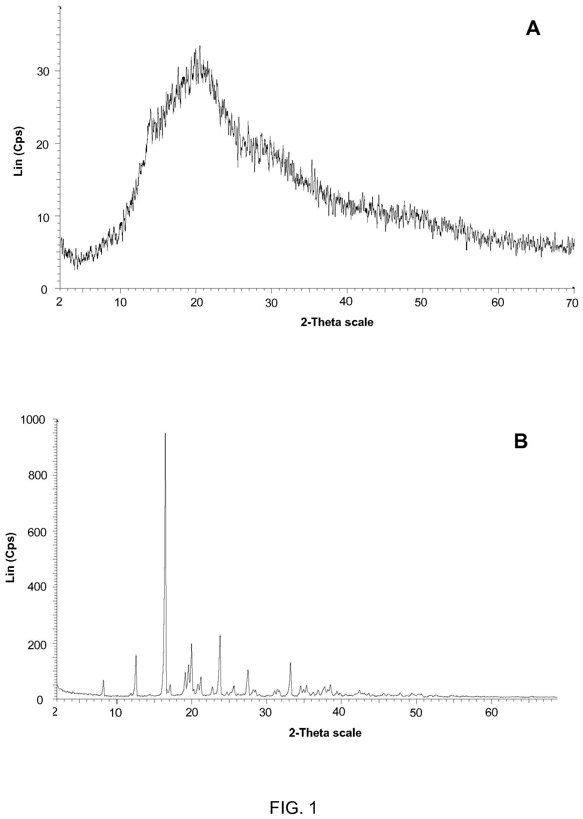

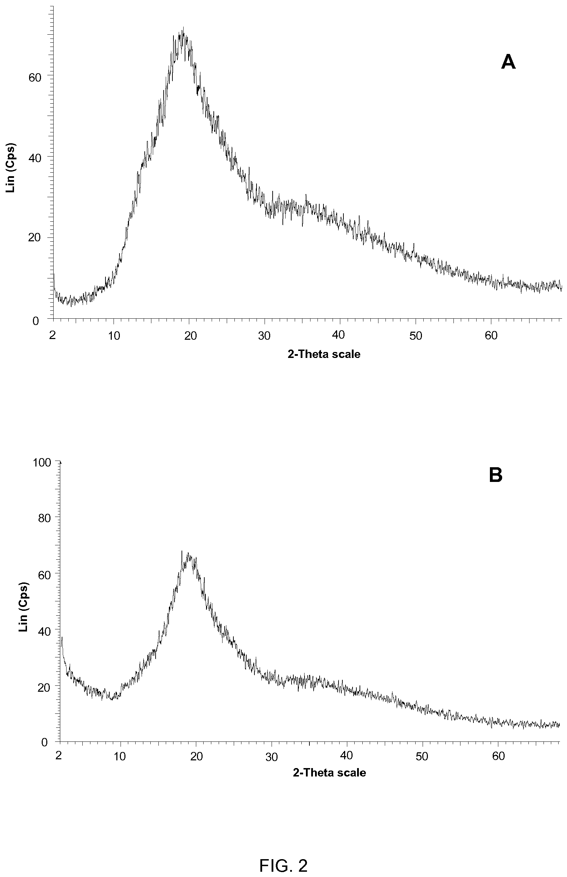

In FIG.1A and 2A, PXRD analysis showed that the spray-drying process permitted to transform both a solution of tiotropium bromide alone and a solution tiotropium bromide-lactose, according to the composition described in example 1, into an amorphous form. However, as shown in FIG 1B, the amorphous spray-dried tiotropium bromide was not stable, as the amorphous form was transformed into crystalline tiotropium during storage. In contrast, as shown in FIG. 2B, the presence of lactose in the spray-dried powders permitted to stabilize tiotropium as a tiotropium -lactose amorphous form even after storage.

### Particle size analysis

Particle size was measured by a technique based on laser light scattering. The volume particle size distribution was measured with a Malvern Mastersizer 2000 ® laser diffractometer using a dry sampling system (Scirocco 2000, Malvern, UK) with a suitable SOP (Standard Operating Procedure).
The particle size distribution is characterized by the mass-volume median diameter (d(0,5)), i.e. the size in microns at which 50 % of the sample is smaller and 50 % is larger, and the mass-volume mean diameter (D[4,3]). Values presented are the average of at least 3 determinations.

Particle size analysis showed that both spray dried formulations obtained from lactose in solution (Example 1) and in suspension (Example 3) presented appropriate particle size properties for deep lung administration.

Indeed, median volume diameters d(0,5) for solution and suspension type formulations were 2.9 µm and 3.0 µm respectively and mean volume diameter D[4,3] for the same formulations were 10.3 µm and 10.5 µm, respectively. Moreover, more than 70% of particles presented particle size below 5.0 µm, which is generally considered as the size limit for good lung penetration.

### Scanning electron microscopy (SEM)

Evaluation of particle size and morphology was achieved by Scanning Electron Microscopy (SEM), using a JSM-610 microscope (Jeol, Japan). Samples were scattered on a thin film of a two-component epoxy resin and then coated with a platinum layer. Acceleration during observation was 25 kV.

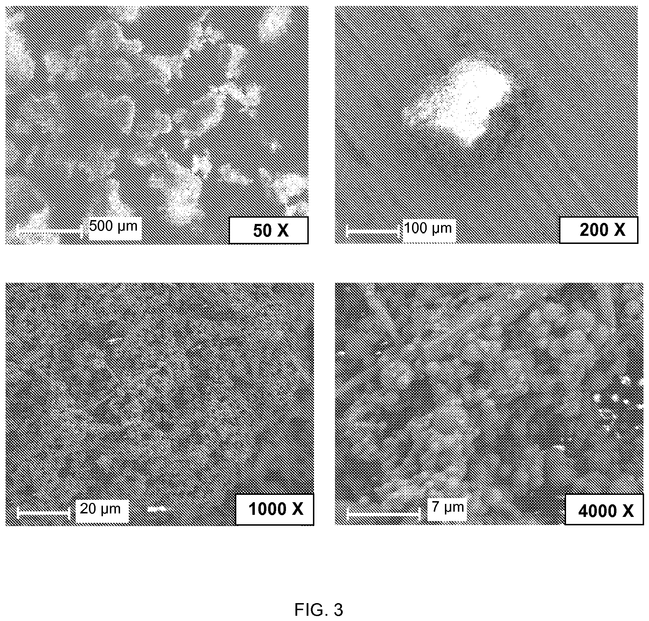

FIG. 3 shows SEM images for spray dried powders obtained from tiotropium-lactose in solution, according to the composition described in example 1 at different magnifications.

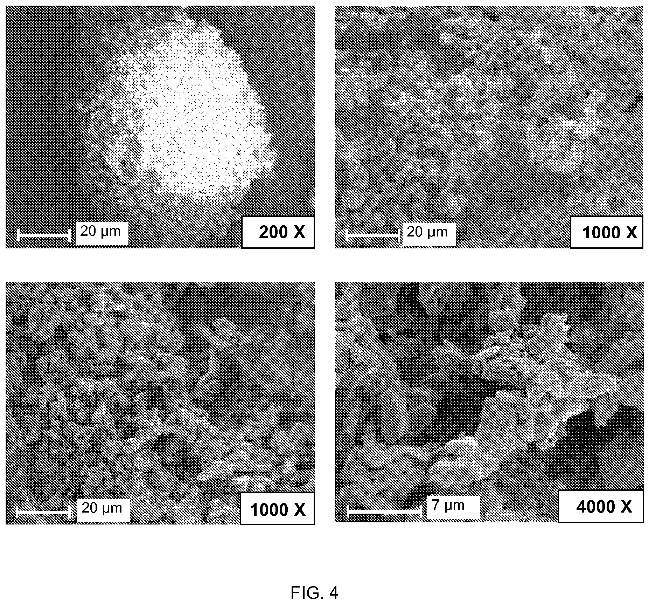

FIG. 4 shows SEM images for spray dried powders obtained from Tiotropium-lactose in suspension, according to the composition described in example 3 at different magnifications.

The morphology and surface structure of the formulations analysed by SEM showed that the spray dried powders consisted of loose agglomerates. The size of agglomerates ranged up to about 100-500 µm. At larger magnifications, we can observe that these agglomerates are composed of small particles of about the micrometer range and which have a more homogeneous spherical-like shape in the case of powder obtained from solutions rather than from suspensions.

### Evaluation of drug distribution homogeneity in spray dried powders and in final Tiotropium - lactose mixtures

The drug distribution homogeneity was evaluated from HPLC, using the method described above.

Results shown in table 2 showed data referred to weighted sample (unit dose in capsule), tiotropium concentration in injected solution from HPLC analysis, amounts of tiotropium in the spray-dried formulations expressed in mg/g and in percentage. These data show a relatively homogeneous drug distribution for two spray-dried batches, showing appropriate drug contents about 7% and 6.7% for spray-dried solutions containing ethanol-water and isopropanol-water.

**Table 2**

| | 9 | mg/l | mg/g | % |
|---|---|---|---|---|
| Example 1 | 0.00506 | 3.6076 | 71.2957 | **7.1** |
| | 0.00500 | 3.5169 | 70.3380 | **7.0** |
| | 0.00500 | 3.2894 | 65.7886 | **6.6** |
| Example 2 | 0.00506 | 3.1509 | 62.2708 | **6.2** |
| | 0.00504 | 3.4112 | 67.6827 | **6.8** |
| | 0.00510 | 3.4286 | 67.2278 | **6.7** |

### In vitro aerodynamic evaluation

The aerodynamic particle size distribution for the new Tiotropium formulations was determined using a Multi-Stage Liquid Impinger (MsLI). A dry powder inhalation device (Fantasmino®) was equipped with a No. 3 HPMC capsule (Capsugel, France). The flow rate was adjusted to a pressure drop of 4 kPa, as typical for inspiration by a patient, resulting in a flow rate of 100 l/min during 2.4 seconds. Five capsules loaded with 5.5 mg powder (18 µg Tiotropium) were taken for each test. Drug deposition in the device, the throat, the four stages and the filter (stage 5) was determined by HighPressure Liquid Chromatography (HPLC) analysis. For accuracy, each test was repeated three times.

The aerodynamic particle size distribution for the reference product Spiriva® was determined using the MsLI with the Handihaler® device and containing gelatine capsules. The flow rate was of 60 l/min during 4 seconds.

The results indicated that the FPD, which roughly corresponds to the drug deposition at stages 3, 4 and the filter (cut-off diameters of 5.27µm, 2.40µm and 1.32µm, respectively), varied within a range of 4.9 µg (FPF of 27%) and 6.4 µg (FPF of 35%) for the developed formulations. These results showed significantly higher FPD values in comparison with the reference product. Indeed, the FPD value obtained for Spiriva® was 1.6 µg that corresponds to a FPF of 8.9%.

**Table 3**

| | **Example 8** | | **Example 9** | | **Example 10** | | **Spiriva** | |
|---|---|---|---|---|---|---|---|---|
| | **Mean** | **S.D.** | **Mean** | **S.D.** | **Mean** | **S.D.** | **Mean** | **S.D.** |
| **Device** | 1.34 | 0.1 | 2.48 | 1.7 | 1.56 | 0.8 | 6.20 | 0.8 |
| **Throat** | 0.77 | 0.2 | 0.48 | 0.2 | 0.37 | 0.4 | 0.07 | 0.1 |
| **1** | 0.57 | 0.5 | 0.38 | 0.4 | 0.24 | 0.2 | 0.00 | 0.0 |
| **2** | 1.26 | 0.2 | 0.97 | 0.1 | 1.29 | 0.2 | 0.09 | 0.2 |
| **3** | 3.21 | 0.7 | 3.22 | 0.3 | 3.89 | 1.1 | 1.48 | 0.6 |
| **4** | 2.28 | 0.2 | 1.68 | 0.3 | 2.15 | 1.9 | 0.15 | 0.1 |
| **Filter** | 0.46 | 0.1 | 0.21 | 0.0 | 0.60 | 0.1 | 0.00 | 0.0 |
| **FPD** | **5.76** | **0.5** | **4.93** | **0.5** | **6.39** | **1.0** | **1.59** | **0.6** |
| **Recovery** | **9.9** | **3.0** | **9.4** | **3.0** | **10.1** | **5.3** | **8.0** | **3.4** |
| **FPF** | **32.0** | **0.6** | **27.4** | **1.3** | **35.5** | **1.0** | **8.9** | **1.1** |

### Preliminary evaluation of the stability of the new formulations

Three mixed coarse monohydrate lactose-tiotropium formulations presenting homogeneity, namely examples 8, 9 and 10, were placed into well closed containers and stored during 15 days and 1 month at 25 °C (room temperature), 40 °C and 60 °C. The tiotropium content of the different samples was evaluated by HPLC analysis as described below at the different storage times and compared to that of the reference product Spiriva®. Results obtained showed that the 3 batches present acceptable stability up to 30 days storage time at the 3 storage temperatures. The Tiotropium content of the new formulations was comparable to that of the reference product.

In table 4 stability values of different formulations at 30 days and at 25 °C, 40 °C and 60 °C are shown. In particular, indication is given on the percentage of the tiotropium content determined by HPLC as well as the standard deviation (S.D.) values.

**Table 4**

| **Stability test at 25 °C** | | | | | | | | |
|---|---|---|---|---|---|---|---|---|
| **Time (days)** | **Reference product** | S.D. | **Example 8** | S.D. | **Example 9** | S.D. | **Example 10** | S.D. |
| **0** | 98.2 | 10.2 | 102.8 | 24.9 | 102.2 | 16.1 | 103.8 | 16.1 |
| **15** | 96.5 | 4.6 | 96.1 | 5.5 | 101.3 | 9.2 | 100.7 | 7.8 |
| **30** | 111.1 | 4.1 | 104.2 | 9.0 | 95.0 | 6.0 | - | - |

| **Stability test at 40 °C** | | | | | | | | |
|---|---|---|---|---|---|---|---|---|
| **Time (days)** | **Reference product** | S.D. | **Example 8** | S.D. | **Example 9** | S.D. | **Example 10** | S.D. |
| **0** | 98.2 | 10.2 | 102.8 | 24.9 | 102.2 | 16.1 | 103.8 | 16.1 |
| **15** | 95.1 | 0.2 | 97.9 | 9.8 | 103.2 | 3.9 | 108.4 | 35.1 |
| **30** | 96.9 | 14.4 | 106.2 | 2.9 | 102.8 | 3.4 | - | - |

| **Stability test at 60 °C** | | | | | | | | |
|---|---|---|---|---|---|---|---|---|
| **Time (days)** | **Reference product** | S.D. | **Example 8** | S.D. | **Example 9** | S.D. | **Example 10** | S.D. |
| **0** | 98.2 | 10.2 | 102.8 | 24.9 | 102.2 | 16.1 | 103.8 | 16.1 |
| **15** | 91.3 | 2.1 | 102.3 | 14.1 | 96.9 | 7.5 | 117.6 | 19.3 |
| **30** | 93.7 | 3.1 | 102.5 | 8.0 | 115.5 | 6.3 | - | - |

## Claims

1. Inhalable particles comprising a stabilized anhydrous amorphous form of tiotropium with a stabilizing agent, which is lactose, wherein the stabilized amorphous form of tiotropium with the stabilizing agent comprises a matrix in which tiotropium is dispersed in a molecular state or wherein the stabilized amorphous form of tiotropium with the stabilizing agent comprises amorphous tiotropium dispersed at the surface of the stabilizing agent, and wherein the particles are mixed with one or more coarse excipients having a mean particle size of 15 to 250 µm.

2. Inhalable particles according to claim 1 , wherein tiotropium comprises tiotropium bromide.

3. Inhalable particles according to any of the claims 1 - 2, having a mean aerodynamic particle size of up to 10 µm.

4. Inhalable particles according to claim 1, wherein the coarse excipient is lactose.

5. A pharmaceutical composition comprising inhalable particles according to any of the claims 1 or 4.

6. The pharmaceutical composition according to claim 5, wherein the tiotropium base content is from 0.02 to 0.8%.

7. The pharmaceutical composition according to any of the claims 5 - 6, in the form of a capsule for inhalation.

8. The pharmaceutical composition according to claim 7, wherein the capsule comprise hydroxypropylmethylcellulose.

9. A process for preparing inhalable particles according to any of the claims 1 - 4, comprising the following steps:
a) dissolving or dispersing the stabilizing agent in a volatile water miscible solvent optionally containing water to form a solution or a suspension;
b) dissolving a tiotropium salt, or a solvate thereof, or any solid form thereof, in a volatile water miscible solvent optionally containing water;
c) mixing the solution of step b) and the solution or suspension of step a);
d) spray-drying the solution/suspension of step c) to obtain the desired particles; and
e) mixing the particles of step d) with one or more coarse excipients having a mean particle size of 15 to 250 µm.

10. The process of claim 9, wherein when in step a) a suspension is formed, the solvent used does not contain water and the stabilizing agent is in the form of particles having a mean particle size of 1 - 9 µm.

11. Inhalable particles as defined in any of the claims 1 - 4 for use in the treatment of asthma or chronic obstructive pulmonary disease.

## Patentansprüche

1. Inhalierbare Partikel, umfassend eine stabilisierte wasserfreie amorphe Form von Tiotropium mit einem Stabilisierungsmittel bei dem es sich um Laktose handelt, wobei die stabilisierte amorphe Form von Tiotropium mit dem Stabilisierungsmittel eine Matrix umfasst, in der das Tiotropium in einem molekularen Zustand dispergiert ist, oder wobei die stabilisierte amorphe Form von Tiotropium mit dem Stabilisierungsmittel amorphes Tiotropium umfasst, das auf der Oberfläche des Stabilisierungsmittels dispergiert ist, und wobei die Partikel mit einem oder mehreren groben Streckmitteln mit einer mittleren Partikelgröße von 15 bis 250 µm vermischt sind.

2. Inhalierbare Partikel nach Anspruch 1, wobei das Tiotropium Tiotropiumbromid umfasst.

3. Inhalierbare Partikel nach einem der Ansprüche 1 bis 2, die eine mittlere aerodynamische Partikelgröße von bis zu 10 µm aufweisen.

4. Inhalierbare Partikel nach Anspruch 1, wobei das grobe Streckmittel Laktose ist.

5. Pharmazeutische Zusammensetzung, die inhalierbare Partikel nach einem der Ansprüche 1 oder 4 umfasst.

6. Pharmazeutische Zusammensetzung nach Anspruch 5, wobei der Gehalt an Tiotropiumbase 0,02 bis 0,8 % beträgt.

7. Pharmazeutische Zusammensetzung nach einem der Ansprüche 5 bis 6 in Form einer Kapsel für die Inhalation.

8. Pharmazeutische Zusammensetzung nach Anspruch 7, wobei die Kapsel Hydroxypropylmethylcellulose umfasst.

9. Verfahren zur Herstellung von inhalierbaren Partikeln nach einem der Ansprüche 1 bis 4, das die folgenden Stufen umfasst:
a) Lösen oder Disperieren des Stabilisierungsmittels in einem flüchtigen mit Wasser mischbaren Lösemittel, das optional Wasser enthält, zur Herstellung einer Lösung oder einer Suspension;
b) Auflösen eines Tiotropiumsalzes oder eines Solvats hiervon oder einer beliebigen festen Form hiervon in einem flüchtigen mit Wasser mischbaren Lösemittel, das optional Wasser enthält;
c) Mischen der Lösung der Stufe b) unter Lösung oder Suspension der Stufe a);
d) Sprühtrocknen der Lösung/Suspension der Stufe c) zur Gewinnung der gewünschten Partikel und
e) Mischen der Partikel der Stufe d) mit einem oder mehreren groben Streckmittein mit einer mittleren Partikelgröße von 15 bis 250 µm.

10. Verfahren nach Anspruch 9, wobei, wenn in Stufe a) eine Suspension hergestellt wird, das verwendete Lösemittel Wasser nicht enthält und das Stabilisierungsmittel in Form von Partikeln mit einer mittleren Partikelgröße von 1 bis 9 µm vorliegt.

11. Inhalierbare Partikel gemäß Definition in einem der Ansprüche 1 bis 4 zur Verwendung bei der Behandlung von Asthma oder chronischer obstruktiver Lungenerkrankung.

## Revendications

1. Particules respirables comprenant une forme amorphe anhydre stabilisée de tiotropium munie d'un agent de stabilisation, qui est le lactose, dans lesquelles la forme amorphe stabilisée de tiotropium munie de l'agent de stabilisation comprend une matrice dans laquelle le tiotropium est dispersé dans un état moléculaire ou dans lesquelles la forme amorphe stabilisée de tiotropium munie de l'agent de stabilisation comprend du tiotropium amorphe dispersé au niveau de la surface de l'agent de stabilisation, et dans lesquelles les particules sont mélangées avec un ou plusieurs excipients grossiers possédant une taille de particule moyenne comprise entre 15 et 250 µm.

2. Particules respirables selon la revendication 1, dans lesquelles le tiotropium comprend du bromure de tiotropium.

3. Particules respirables selon l'une quelconque des revendications 1 à 2 possédant une taille de particule aérodynamique moyenne maximale de 10 µm.

4. Particules respirables selon la revendication 1, dans lesquelles l'excipient grossier est le lactose.

5. Composition pharmaceutique comprenant les particules respirables selon l'une quelconque des revendications 1 ou 4.

6. Composition pharmaceutique selon la revendication 5, dans laquelle la teneur en base de tiotropium est comprise entre 0,02 et 0,8 %.

7. Composition pharmaceutique selon l'une quelconque des revendications 5 à 6, sous la forme d'une capsule destinée à l'inhalation.

8. Composition pharmaceutique selon la revendication 7, dans laquelle la capsule comprend de l'hydroxypropylméthylcellulose.

9. Processus de préparation de particules respirables selon l'une quelconque des revendications 1 à 4, comprenant les étapes suivantes consistant à :
a) dissoudre ou disperser l'agent de stabilisation dans un solvant volatile miscible dans l'eau contenant éventuellement de l'eau pour former une solution ou une suspension ;
b) dissoudre un sel de tiotropium, où un solvate de celui-ci, ou toute forme solide de celui-ci, dans un solvant volatile miscible dans l'eau contenant éventuellement de l'eau ;
c) mélanger la solution de l'étape b) et la solution ou la suspension de l'étape a) ;
d) sécher par atomisation la solution/suspension de l'étape c) pour obtenir les particules souhaitées ; et
e) mélanger les particules de l'étape d) avec un ou plusieurs des excipients grossiers possédant une particule de taille moyenne comprise entre 15 et 250 µm.

10. Processus selon la revendication 9, dans lequel dans l'étape a) une suspension est formée, le solvant utilisé ne contient pas d'eau et l'agent de stabilisation est sous la forme de particules possédant une taille de particule moyenne comprise entre 1 et 9 µm.

11. Particules respirables telles que définies selon l'une quelconque des revendications 1 à 4 pour une utilisation dans le traitement de l'asthme ou d'une maladie pulmonaire obstructive chronique.
